Europäisches Patentamt

(19) European Patent Office

Office européen des brevets

(11) Numéro de publication : **0 111 796
B1**

(12) **FASCICULE DE BREVET EUROPÉEN**

(45) Date de publication du fascicule du brevet :
28.05.86

(51) Int. Cl.⁴ : **A 61 M   5/20**, A 61 M   5/28

(21) Numéro de dépôt : **83112127.2**

(22) Date de dépôt : **02.12.83**

(54) **Ampoule-seringue.**

(30) Priorité : **20.12.82 PCT/CH82/00134**

(43) Date de publication de la demande :
**27.06.84 Bulletin 84/26**

(45) Mention de la délivrance du brevet :
**28.05.86 Bulletin 86/22**

(84) Etats contractants désignés :
**AT BE CH DE FR GB IT LI LU NL SE**

(56) Documents cités :
**DE-A- 2 008 751
DE-C-   139 640
DE-C-   569 851
FR-A-   714 108
FR-A- 1 484 784
FR-A- 1 567 814
FR-A- 2 330 415
GB-A-    13 142
US-A- 2 971 509**

(73) Titulaire : **MEDITEC S.A.
11, Boulevard du Prince Henri
L-2014 Luxembourg (LU)**

(72) Inventeur : **Meyer, Gabriel
10A, chemin des Princes
CH-1222 Vesenaz (CH)**
Inventeur : **Howald, Ernst
10B, chemin des Princes
CH-1222 Vesenaz (CH)**

(74) Mandataire : **Nithardt, Roland
CABINET ROLAND NITHARDT Rue Edouard Verdan
15
CH-1400 Yverdon (CH)**

EP 0 111 796 B1

Jouve, 18, rue St-Denis, 75001 Paris, France

## Description

La présente invention concerne une ampoule-seringue comprenant une ampoule préremplie d'un liquide, notamment d'un médicament à injecter, ladite ampoule étant pourvue à son extrémité ouverte d'un organe d'obturation et de distribution mobile axialement par rapport à l'ampoule entre une première position dite d'obturation et une seconde position dite d'injection, cet organe comportant à son extrémité externe un embout porte-aiguille et étant traversé par un conduit reliant ledit embout à l'intérieur de l'ampoule lorsque ledit organe se trouve dans sa position d'injection, ledit conduit comprenant une branche axiale débouchant à l'extérieur du côté de l'embout porte-aiguille et une branche transversale communiquant avec ladite branche axiale, la ou les extrémités ouvertes de ladite branche transversale débouchant à l'intérieur de l'ampoule.

On connaît déjà des ampoules-seringues de ce type décrites notamment dans le brevet français FR-A-714 108. Ces dispositifs se composent d'une ampoule équipée d'un bouchon d'obturation serti à l'extrémité ouverte de l'ampoule, et associé à une vanne rotative ou à mouvement axial traversée par un conduit axial, raccordé à un conduit radial destiné à déboucher dans une cavité qui se trouve en communication avec l'intérieur de l'ampoule. Dans l'exemple illustré par les figures 1 et 2 de ce brevet, l'ouverture et la fermeture de la vanne s'obtiennent par rotation du porte-aiguille, dans l'exemple illustré par la figure 3 elles s'obtiennent par mouvement axial vis-à-vis de l'ampoule.

La fabrication de l'ampoule-seringue décrite est extrêmement coûteuse et le produit ne donne pas satisfaction à de nombreux points de vue. En effet, cette construction comporte un bouchon d'obturation en contact avec le produit pharmaceutique contenu dans l'ampoule, une vanne rotative ou à mouvement axial logée dans un alésage central ménagé dans le bouchon d'obturation et également partiellement en contact avec le produit à injecter, un capuchon porte-aiguille spécial adapté au bouchon d'obturation et un ressort spirale placé entre le bouchon et le capuchon.

La fabrication d'une aiguille et d'un embout porte-aiguille spéciaux, constitue une opération coûteuse. Le fait que l'aiguille soit montée sur l'embout porte-aiguille au moment de l'assemblage des pièces et surtout au moment de la mise sous pression de l'ampoule, implique que le gaz sous pression à introduire dans l'ampoule soit injecté à travers la seringue, ce qui nécessite un appareillage compliqué pour éviter un endommagement de l'aiguille. La surpression pourrait également être obtenue en faisant le remplissage dans une enceinte pressurisée, mais l'opération est compliquée et son coût de revient élevé. La mise sous pression du liquide pharmaceutique de l'appareil peut être effectuée par injection de gaz à travers l'embout porte-aiguille, préalablement mis en place, suivie d'une soudure ou d'un collage de l'aiguille. Les manipulations de l'aiguille sont coûteuses parce qu'elles nécessitent des précautions particulières. Elles doivent se faire dans des conditions stériles et exclure toute souillure, en particulier par des résidus de colle.

Le médicament est en contact avec trois éléments réalisés en des matériaux différents, l'ampoule, le bouchon et la vanne. En outre, l'étanchéité entre le bouchon et la vanne est difficile à obtenir à moins que le corps de cette vanne soit fortement serré à l'intérieur du bouchon, ce qui implique un ressort puissant et contrarie le recul de l'ampoule au moment de l'injection.

Le maintien en place du bouchon nécessite une bague de sertissage. Les pièces composant le dispositif d'obturation de l'ampoule sont les suivantes : un bouchon, une vanne logée dans une cavité cylindrique de ce bouchon, une bague de sertissage, un capuchon porte-aiguille, un ressort et une aiguille. La fabrication de toutes ces pièces et leur montage sont excessivement coûteux. Par ailleurs, la préparation de la seringue en vue de son utilisation requiert les opérations suivantes consistant à remplir l'ampoule de médicament, à mettre en place le bouchon sur cette ampoule, à fixer le bouchon au moyen d'une bague de sertissage, à mettre en place la vanne dans l'alésage central du bouchon, à mettre en place le ressort, à injecter un gaz comprimé dans l'ampoule pour mettre le liquide sous pression et à fixer l'aiguille. Ces différentes phases préparatoires sont à la fois délicates et coûteuses.

D'autre part, on sait par l'article paru en page 17, de l'ouvrage « Récipients en matière plastique pour les préparations pharmaceutiques, essai et contrôle », publié en 1974 par l'Organisation Mondiale de la Santé, par Jack Cooper, que les récipients composites sont souvent propices aux interactions entre les matériaux. En particulier, on constate parfois une migration de certains composants de la matière synthétique dans le liquide médicamenteux. Les interactions entre le contenu et le contenant, lorsque ce dernier est un polymère, peuvent modifier les propriétés mécaniques de ce polymère. Même si ces propriétés sont satisfaisantes à l'origine, les changements résultant d'interactions survenant progressivement obligent les fabricants à pratiquer des essais de stabilité en cours du stockage.

On connaît encore bien d'autres ampoules-seringues préremplies comportant une ampoule contenant un médicament liquide sous pression et un organe d'obturation qui obture cette ampoule jusqu'au moment de son utilisation. Un document illustrant particulièrement bien une des techniques couramment utilisées à ce jour est le brevet britannique GB-A-13 142 qui décrit une ampoule-seringue préremplie qui est obturée par un bouchon étanche destiné à être percé par une aiguille double dont une extrémité pénètre à travers le bouchon à l'intérieur de l'ampoule et dont l'autre extrémité sert à l'injection du médicament dans le corps du patient. La mise sous pression d'une telle ampoule doit se faire automatiquement à l'intérieur d'une enceinte pressurisée car l'obturateur n'a d'effet que dans un sens. L'aiguille dont la forme est particulière nécessite une construction spéciale relativement coûteuse, les aiguilles standard étant inutilisables avec ce dispositif. Enfin le perçage d'un bouchon en élastomère par la double aiguille peut provoquer la migration de particules inertes entraînées par le liquide médicamenteux dans le corps du patient et présente un réel danger pour celui-ci.

La présente invention se propose de pallier les différents inconvénients relatés ci-dessus et d'offrir au personnel soignant un instrument utile, facile à manipuler, de construction économique et présentant pour le patient toutes les garanties d'hygiène et de propreté requises.

Dans ce but, l'ampoule-seringue préremplie selon l'invention est caractérisée en ce que l'ampoule présente à son extrémité ouverte un col de section rétrécie par rapport à son corps, en ce que l'organe d'obturation est disposé à l'intérieur dudit col en contact direct avec sa paroi interne, cet organe ayant une forme sensiblement complémentaire et des dimensions sensiblement égales à celles dudit col et en ce que la branche transversale dudit conduit est ménagée dans l'organe d'obturation en position telle que son ou ses extrémités débouchant à l'intérieur de l'ampoule soient obturées par la paroi interne du col de l'ampoule lorsque l'organe d'obturation se trouve dans ladite position d'obturation.

La présente invention, ses caractéristiques et ses principaux avantages seront mieux compris en référence à la description d'exemples de réalisation et au dessin annexé dans lequel :

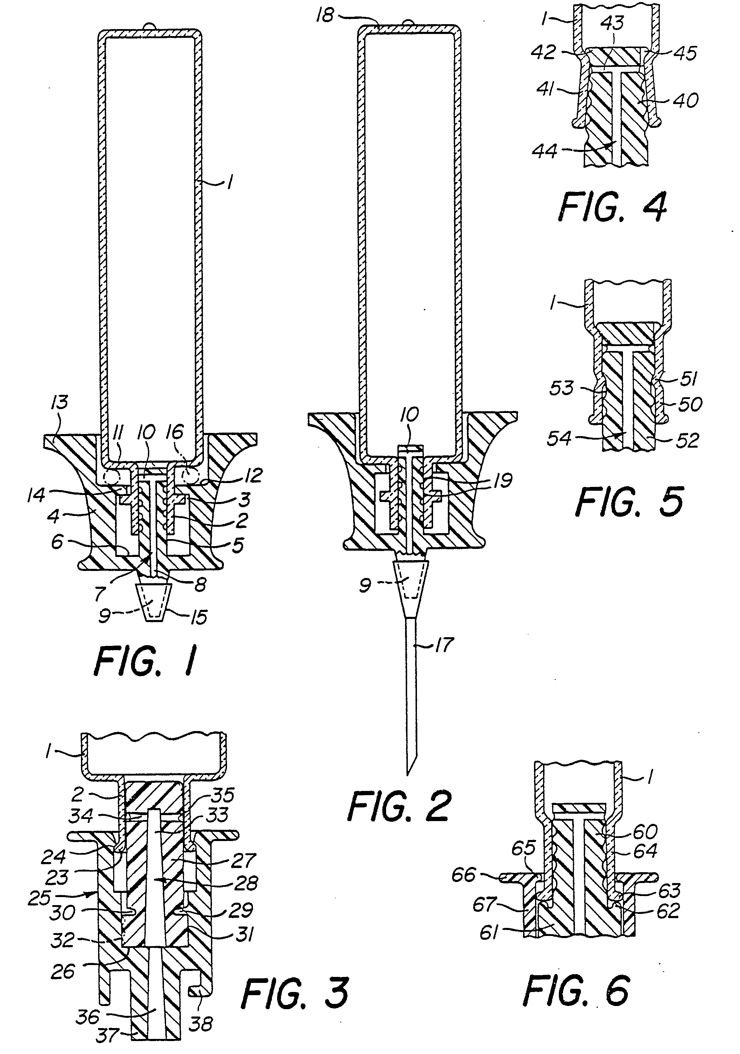

La figure 1 illustre schématiquement une première forme de réalisation de l'ampoule-seringue préremplie selon l'invention, représentée dans sa position de stockage.

La figure 2 représente l'ampoule-seringue selon la fig. 1 dans sa position d'utilisation.

La figure 3 est une vue partielle agrandie représentant essentiellement l'organe d'obturation et la capsule associée à cet organe.

Les figures 4, 5 et 6 représentent diverses autres formes de réalisation de l'organe d'obturation associé à l'ampoule-seringue préremplie selon l'invention.

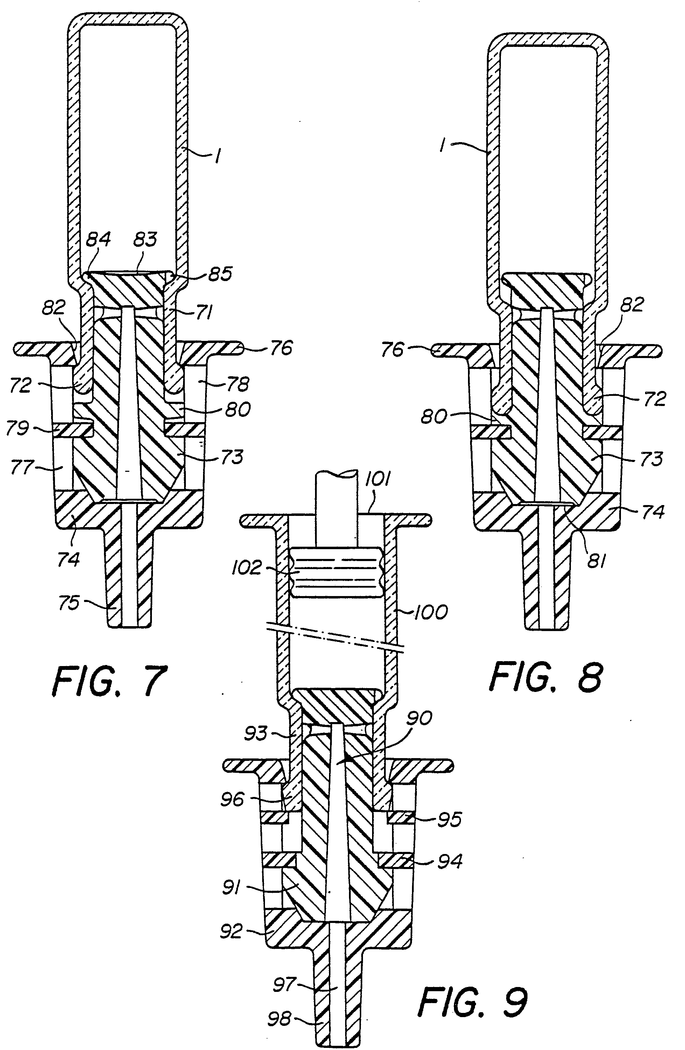

La figure 7 illustre une variante de l'ampoule-seringue selon la fig. 3, représentée dans sa position de repos.

La figure 8 illustre l'ampoule-seringue de la fig. 7 dans sa position d'utilisation.

La figure 9 illustre une autre forme de réalisation de l'ampoule-seringue préremplie selon l'invention, représentée dans sa position de stockage, et dans laquelle l'ampoule à gaz sous pression a été remplacée par un corps de seringue ouvert à piston.

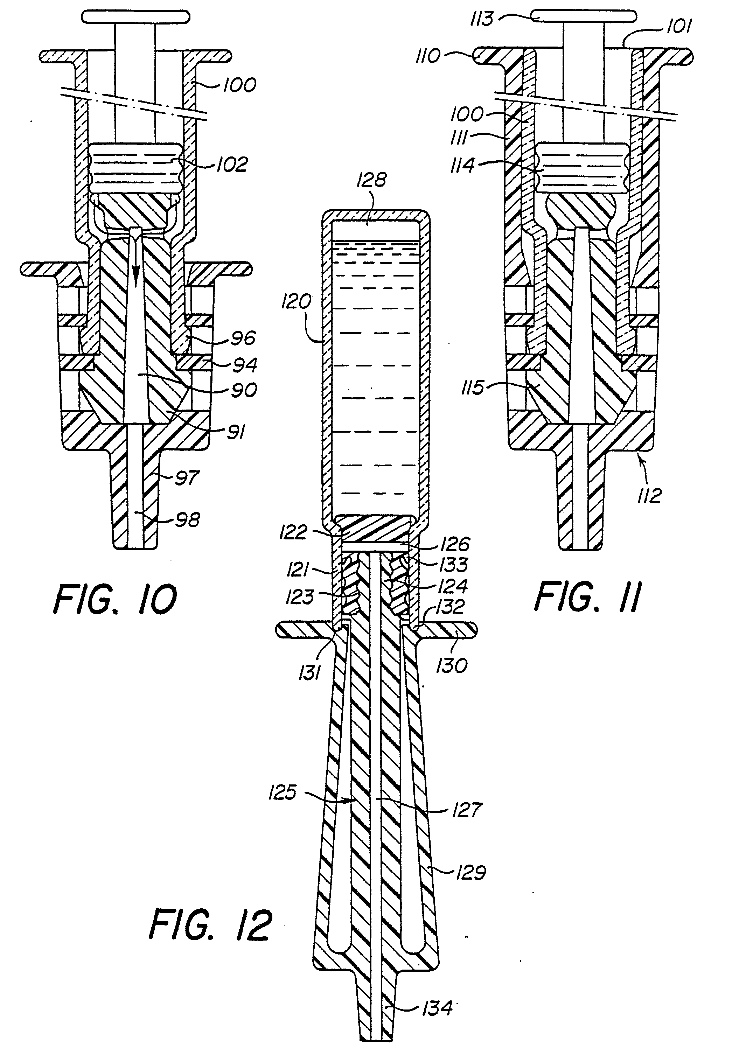

La figure 10 représente l'ampoule-seringue selon la fig. 9 dans sa position d'injection.

La figure 11 illustre une autre forme de réalisation de l'ampoule-seringue préremplie selon la fig. 9, représentée dans sa position d'utilisation.

La figure 12 illustre une variante de l'ampoule-seringue préremplie selon l'invention, dans sa position de stockage, le liquide médicamenteux étant placé sous vide partiel à l'intérieur de l'ampoule.

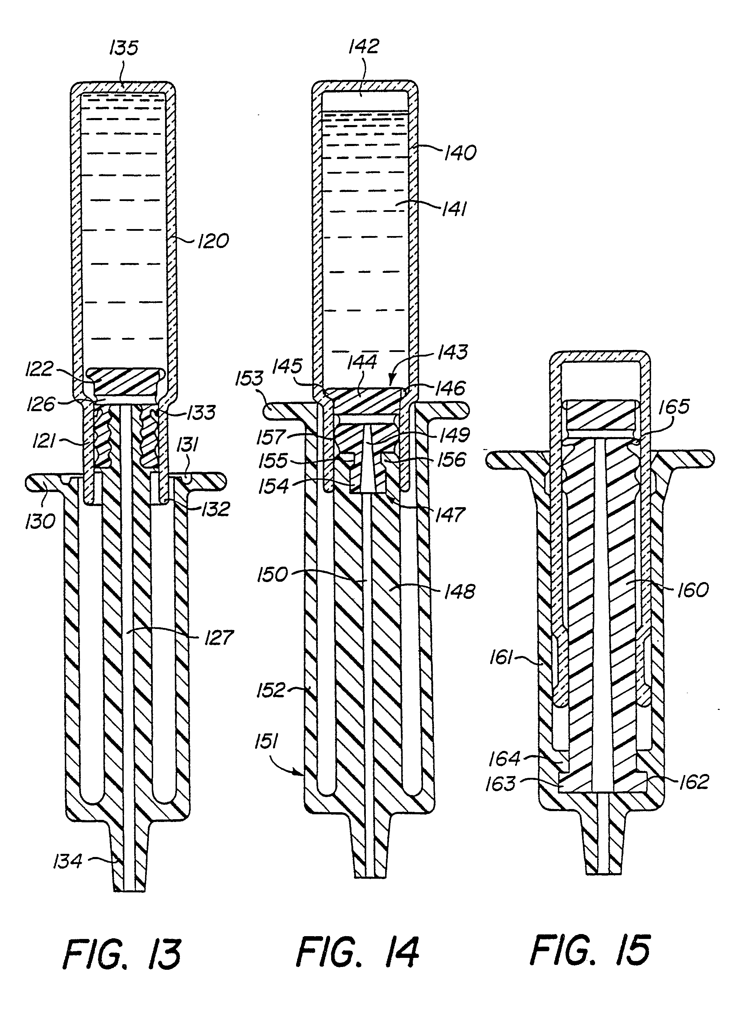

La figure 13 représente l'ampoule-seringue préremplie de la fig. 12 dans sa position d'injection.

La figure 14 illustre une autre forme de réalisation de l'ampoule-seringue selon l'invention, dans laquelle le médicament liquide est disposé dans l'ampoule avec un peu de gaz inerte sous pression atmosphérique ou éventuellement en légère surpression.

La figure 15 illustre une variante du dispositif de la fig. 14, dans laquelle l'organe d'obturation est réalisé d'une pièce avec la tige du piston.

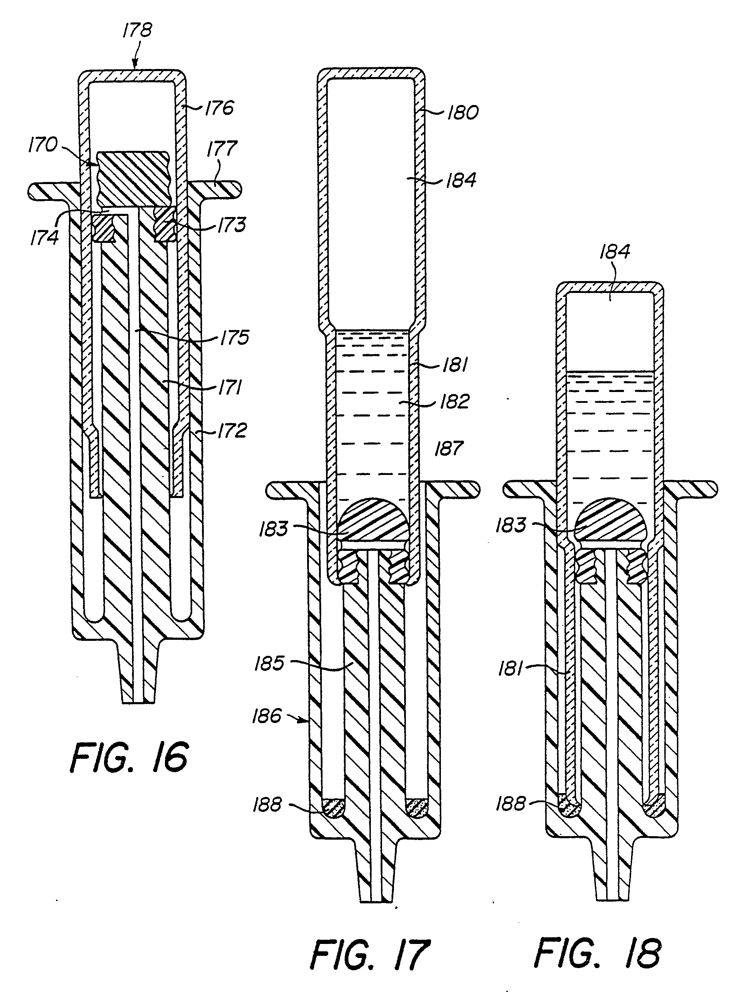

La figure 16 illustre une autre variante de l'ampoule-seringue de la fig. 14.

Les figures 17 et 18 représentent une forme de réalisation de l'ampoule-seringue préremplie selon l'invention, dans laquelle le liquide médicamenteux est surmonté d'un gaz détendu, respectivement en position de stockage et en position d'utilisation.

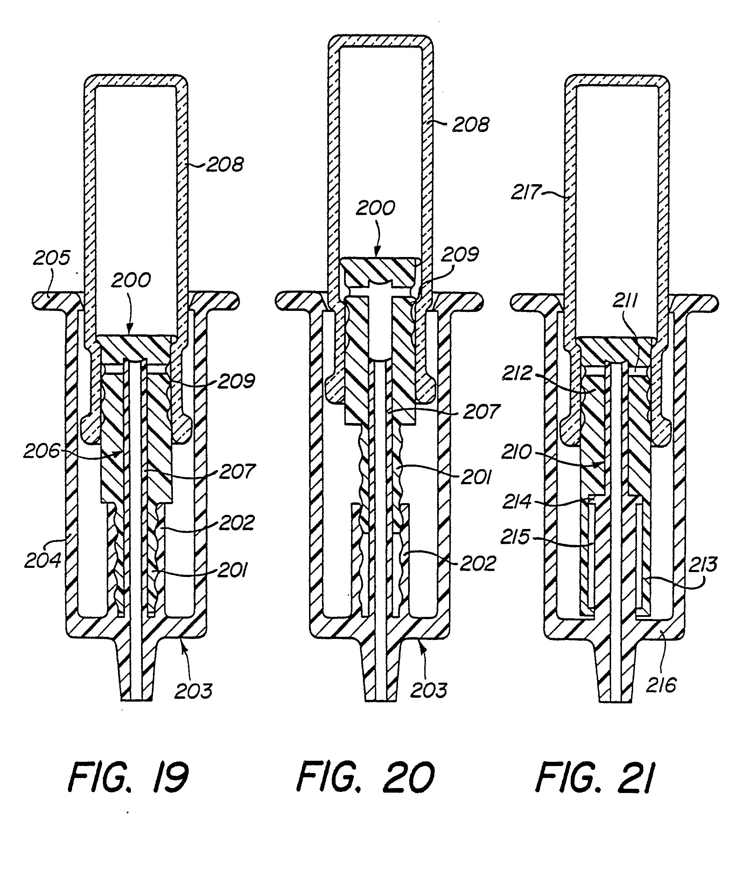

Les figures 19 et 20 illustrent une autre forme de réalisation de l'ampoule-seringue préremplie selon l'invention, respectivement en position de stockage et en position d'utilisation.

La figure 21 illustre un autre exemple d'ampoule-seringue préremplie représentée dans sa position de stockage.

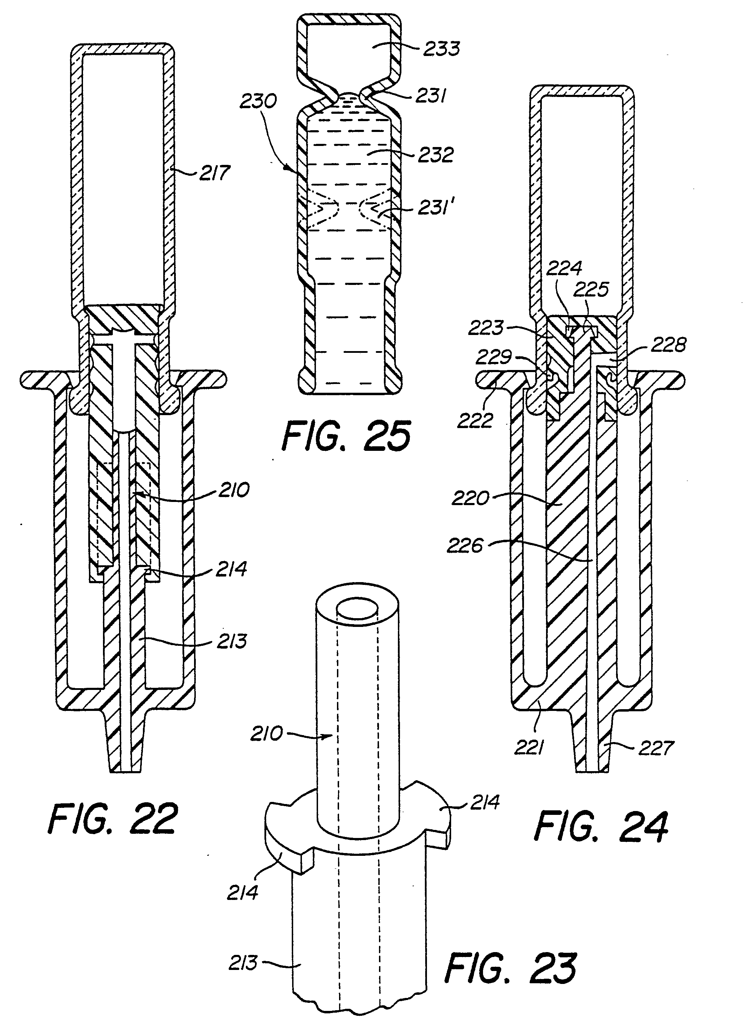

La figure 22 illustre l'ampoule-seringue préremplie de la fig. 21 dans sa position d'utilisation.

La figure 23 représente une vue en perspective de la pièce télescopique montée à l'intérieur du conduit central de l'organe d'obturation.

La figure 24 illustre une autre forme de réalisation de l'ampoule-seringue selon l'invention, et

La figure 25 illustre une forme de réalisation particulièrement avantageuse de l'ampoule contenant un médicament liquide et un gaz sous pression.

L'ampoule-seringue préremplie selon les figures 1 et 2 comporte une ampoule 1 pourvue d'un col cylindrique 2, de section rétrécie par rapport à son corps et munie d'un orifice. Ce col est pourvu d'un bourrelet phériphérique 3 qui coopère avec un bourrelet annulaire 14 ménagé sur les parois intérieures d'une capsule creuse 4 adaptée au col 2 de l'ampoule 1. Cette capsule est solidaire de l'organe d'obturation 5 engagé à l'intérieur du col 2 et fixé au fond 6 de la capsule de manière à se dresser perpendiculairement à ce fond, dans la cavité intérieure à la capsule. L'organe d'obturation 5 comporte un conduit intérieur 7 en forme de T dont la branche axiale centrale 8 débouche dans un embout porte-aiguille 9, et dont la branche transversale 10 débouche contre les parois intérieures du col 2. Pendant le stockage de l'ampoule-seringue préremplie, l'embout porte-aiguille 9 est recouvert d'un capuchon de protection 15. La jonction entre le corps de l'ampoule 1 et son col définit un épaulement annulaire 11 qui correspond à un épaulement 12 localisé entre des ailettes de maintien 13 qui équipent la capsule 4 à son extrémité opposée au fond 6, et entre le bourrelet annulaire 14. Un organe élastique, tel qu'une bague torique 16 peut être interposé entre l'épaulement 11 de l'ampoule et l'épaulement 12 de la capsule pour éviter que l'ampoule passe accidentellement de sa position de stockage illustrée par la fig. 1 à sa position d'utilisation représentée par la fig. 2.

Dans cette dernière figure, la bague torique a été retirée à travers une ouverture appropriée ménagée dans la capsule ou écrasée sous l'effet d'une pression exercée sur le fond de l'ampoule 1. Au préalable, le capuchon 15 a été retiré de l'embout porte-aiguille et remplacé par une aiguille 17. La pression exercée sur le fond 18 du corps de l'ampoule 1 a pour effet de rapprocher les deux épaulements 11 et 12 et de dégager les ouvertures latérales de la branche radiale 10 du conduit intérieur 7. Le liquide contenu à l'intérieur de l'ampoule et maintenu sous pression, peut s'écouler librement à travers ce conduit et être injecté au moyen de l'aiguille 17 dans le corps du patient. La présence de la bague élastique permet de ramener l'ampoule dans sa position initiale et d'arrêter l'injection à n'importe quel moment.

Ainsi, grâce à cette réalisation, le liquide médicamenteux n'est en contact, pendant son stockage, qu'avec la matière dans laquelle est réalisé l'organe d'obturation 5, et avec les parois du corps de l'ampoule 1. Cette matière est un élastomère ou tout autre matériau approprié ayant des caractéristiques élastiques. L'étanchéité de l'ampoule est assurée d'une manière parfaite entre les parois rigides du col de l'ampoule et les parois périphériques souples de l'organe d'obturation. Pour améliorer cette étanchéité, l'organe d'obturation comporte un certain nombre de bourrelets périphériques 19 qui forment autant de joints étanches entre les deux surfaces en contact.

La fig. 3 illustre une variante de réalisation présentant l'avantage d'une fabrication particulièrement simple. L'ampoule-seringue préremplie représentée comporte comme précédemment une ampoule 1 pourvue d'un col cylindrique 2. Ce col comporte à son extrémité adjacente à son orifice un bourrelet 23 constituant un rebord, qui coopère avec un bourrelet 24 pour assurer la retenue de la capsule 25 sur l'ampoule 1. Le fond 26 de la capsule 25 porte un organe d'obturation 27 pourvu d'un conduit central 28 en forme de T. Dans l'exemple représenté, l'organe d'obturation est rapporté et fixé au fond de la capsule par divers moyens. Sur la partie droite de la figure, la capsule porte sur sa paroi intérieure un bourrelet 29, de préférence de forme annulaire qui s'engage dans une rainure annulaire 30 de forme complémentaire pour assurer le couplage de l'organe d'obturation 27 et de la capsule 25. Sur la partie gauche de la figure, ce couplage se fait par rabattement de matière par soudure aux ultrasons pour former un bourrelet 29. Cette liaison pourrait également être faite par un double filetage 31 et 32 représenté en traits interrompus.

Le conduit intérieur 28 se compose d'une branche axiale de forme tronconique 33 et d'une branche transversale 34 dont les ouvertures latérales débouchent dans un petit évidement 35 délimité par la paroi du col de l'ampoule et par les parois de l'obturateur entourant ces ouvertures. La branche axiale 33 est prolongée par un conduit axial 36 traversant le fond 26 de la capsule 25 et l'embout porte-aiguille 37. Un bourrelet de retenue 38 peut équiper la capsule 25 pour bloquer l'aiguille en position sur l'embout porte-aiguille 37.

La fig. 4 illustre une autre forme de réalisation dans laquelle l'organe d'obturation 40 présente une forme tronconique qui correspond à la forme tronconique du col 41 de l'ampoule 1. L'organe d'obturation comporte à son extrémité intérieure à l'ampoule 1 un bourrelet de retenue 42 dont le diamètre est supérieur au diamètre minimal du corps de l'organe d'obturation dans la zone où débouche la branche transversale 43 du conduit intérieur 44. Le diamètre de ce bourrelet de retenue 42 est cependant inférieur au diamètre intérieur de l'ampoule pour permettre un écoulement du liquide pharmaceutique à travers le conduit 44 lorsque l'organe d'obturation est repoussé vers l'intérieur de cette capsule. A défaut de restriction du diamètre du bourrelet 42 par rapport à celui du corps de l'ampoule 1, le bourrelet 42 pourrait présenter une ou plusieurs entailles axiales 45 permettant également l'écoulement du liquide lorsque l'organe d'obturation se trouve dans sa position d'injection. La conicité de l'organe d'obturation lui confère l'élasticité nécessaire pour permettre le recul automatique au moment du remplissage de l'ampoule et de sa mise sous pression, et lors de l'utilisation de l'ampoule-seringue pour interrompre l'injection.

La fig. 5 illustre une variante selon laquelle le col 50 de l'ampoule 1 est cylindrique et comporte un bourrelet intérieur 51, de préférence de forme annulaire, mais qui pourrait également être remplacé par des bossages discontinus. L'organe d'obturation 52 comporte une rainure annulaire 53 qui coopère avec le bourrelet 51 pour assurer d'une part l'arrêt de l'organe d'obturation en position de stockage et le rappel élastique de cet organe pour le remplissage et la mise sous pression de l'ampoule ou pour permettre l'interruption de l'injection. Comme pour les autres réalisations décrites ci-dessus, l'organe d'obturation 52 comporte un conduit intérieur 54 en forme de T et le col 50 a un diamètre intérieur inférieur à celui du corps de l'ampoule 51.

La figure 6 diffère de la réalisation de la fig. 5 en ce que l'organe d'obturation 60 comporte une zone élargie 61 au voisinage de son extrémité inférieure. Cette zone définit un renflement 62 élastique qui prend appui sur un rebord 63 entourant l'ouverture du col 64 de l'ampoule 1. Ce renflement constitue un organe de rappel permettant de ramener l'organe d'obturation 60 dans sa position de stockage à la fin du remplissage de l'ampoule ou au moment de l'interruption de l'injection en cours de l'utilisation de l'ampoule-seringue. Le blocage en position de stockage est obtenu par la coopération du rebord 63 avec un bourrelet intérieur 65 équipant les ailettes 66 de la capsule 67.

La fig. 7 représente une ampoule-seringue préremplie comportant une ampoule 1 pourvue d'un col 71 de section rétrécie dont le bord est équipé d'un renflement 72, un organe d'obturation 73 partiellement engagé à l'intérieur de ce col et une capsule 74 liée à l'organe d'obturation 73 et portant à son extrémité inférieure un embout porte-aiguille 75 et à son extrémité supérieure deux ailettes latérales 76. Contrairement à l'exemple illustré par la fig. 3, dont la capsule réalisée d'une pièce comporte des parois latérales pleines, la capsule de ce dispositif comporte des parois latérales pourvues d'ouvertures latérales 77 et 78 séparées par une pièce annulaire 79 ou en forme de secteurs annulaires destinés à assurer la fixation de l'organe d'obturation dans la cavité centrale de la capsule 74. L'insertion de l'organe d'obturation 73 dans la capsule 74 s'obtient grâce à l'élasticité de la matière. L'organe d'obturation comporte avantageusement un bourrelet périphérique 80 élastique qui s'écrase, comme le montre la fig. 8 lorsque l'ampoule 1 est poussée vers le bas dans le sens de la flèche A par rapport aux ailettes latérales 76 de la capsule 74. Ce bourrelet élastique joue en outre le rôle d'un ressort de rappel qui tend à ramener l'ampoule 1 dans sa position initiale lorsque l'utilisateur relâche la pression qu'il exerce sur le fond de cette ampoule. Sous l'effet de cette pression, l'organe d'obturation 73 s'écrase à sa base contre le fond 81 de la capsule et augmente l'étanchéité entre ces deux composants. Chacune des ailettes 76 comporte le long de son bord intérieur une protubérance 82 destinée à coopérer avec le bourrelet 72 entourant l'extrémité du col de la capsule 1 pour assurer la retenue de cette capsule pendant le stockage de l'ampoule-seringue préremplie.

Comme sur le dispositif représenté notamment par les figures 4 et 5, l'organe d'obturation 73 comporte à son extrémité supérieure 83 un bourrelet 84 qui s'écrase sous l'effet de la pression régnant dans l'ampoule et qui assure une bonne étanchéité avec les parois de l'ampoule 1. Lorsque l'organe d'obturation est repoussé dans le sens opposé à la flèche A, l'extrémité supérieure de l'organe d'obturation se détend et le liquide peut s'écouler par une entaille périphérique 85 ménagée dans le bourrelet 84.

Alors que toutes les ampoules-seringues préremplies décrites précédemment comportaient une ampoule fermée dans laquelle le liquide pharmaceutique est stocké avec un gaz inerte sous pression, l'ampoule-seringue selon les figures 9 et 10 comporte un corps de seringue 100 dont le fond 101 est ouvert pour permettre l'introduction d'un piston 102 destiné à refouler un liquide pharmaceutique à travers le conduit intérieur 90 en forme de T de l'organe d'obturation 91, associé à la capsule 92. Le dispositif d'obturation composé de l'organe d'obturation 91 et de la capsule 92 présente des similitudes avec le dispositif d'obturation composé de l'organe d'obturation 73 et de la capsule 74 illustré par les figures 7 et 8. Toutefois, dans cet exemple, la capsule 92 comporte deux éléments annulaires 94 et 95 ou en forme de secteurs annulaires qui constituent le premier des moyens de verrouillage de l'organe d'obturation 91 et le second une butée d'arrêt qui coopère avec le rebord 96 équipant le col 93 du corps 100 de la seringue. Sur la fig. 9, le rebord 96 est bloqué par l'organe de retenue 95 et l'extrémité supérieure de l'organe d'obturation 91 obture le col 93 de la seringue de sorte qu'aucun liquide contenu dans le corps de cette dernière ne puisse s'écouler à travers le conduit central 90 en forme de T qui communique avec le conduit axial 97 de l'embout porte-aiguille 98. Sur la fig. 10, le corps de la seringue 100 a été repoussé de telle manière que le rebord 96 vienne en butée contre la face supérieure de l'organe de retenue annulaire 94 ou en forme de secteurs annulaires, ce qui a pour effet de dégager les ouvertures latérales de la branche transversale du conduit central 90. Le piston 102 a été repoussé vers le bas en direction de l'organe d'obturation 91 de manière à repousser le médicament à travers le conduit 90 en direction du conduit 97 traversant l'embout porte-aiguille 98.

La fig. 11 représente une variante du dispositif illustré par les figures 9 et 10. Cette réalisation ne diffère de celle des figures 9 et 10 que par la forme et la disposition des ailettes latérales 110. Ces ailettes 110 sont de préférence fixées à l'extrémité supérieure d'une tige 111 directement liée au bord supérieur de la capsule 112. Elles sont avantageusement au nombre de deux, ce qui permet à l'opérateur de retenir le corps 100 de la seringue au moyen de l'index et du majeur et d'appuyer à l'aide du pouce sur la tige 113 du piston 114. Il est bien entendu que ces deux ailettes pourraient être remplacées par un manchon cylindrique prolongeant la capsule 102 sur toute la longueur du corps de la seringue et comportant à son extrémité supérieure un rebord annulaire perpendiculaire à l'axe de ce manchon. Pour amener le corps de la seringue et l'organe d'obturation 115 dans leur position relative d'utilisation telle que représentée par la figure, il suffit à l'opérateur d'appuyer sur le fond 101 du corps 100 de la seringue en maintenant la capsule par les ailettes 110 qui y sont attachées.

Les figures 12 et 13 illustrent une autre forme de réalisation de l'ampoule-seringue décrite précédemment comportant dans ce cas une ampoule 120 fermée à une de ses extrémités et obturée au niveau de son col 121 de section rétrécie par rapport à l'ampoule 120, par un organe d'obturation 122 qui sert également de piston. Cet organe d'obturation comporte une cavité intérieure 123 dans laquelle est engagée l'extrémité 124 d'une tige de piston 125. Le conduit central d'écoulement de la solution pharmaceutique contenue dans l'ampoule 120 comporte dans ce cas une branche transversale 126 ménagée dans l'organe d'obturation 122 et une branche axiale 127 raccordée à la branche radiale 126 et traversant de part en part la tige de piston 125. L'ampoule 120 contient une solution pharmaceutique qui ne remplit pas complètement cette ampoule de telle manière qu'il subsiste un espace vide 128. Pour éviter la pénétration de l'organe d'obturation 122 à l'intérieur de l'ampoule 120, sous l'effet de l'aspiration due au vide créé dans cette ampoule au moment de son remplissage, la tige de piston solidaire de l'organe d'obturation 122 est associée à deux bras latéraux 129 dont les extrémités supérieures munies d'ailettes 130 comportent des encoches 131 dans lesquelles peut s'engager le bord 132 du col 121 de l'ampoule 120. Une languette périphérique 133 est ménagée tout autour de l'organe d'obturation 122 en dessous des embouchures de la branche transversale 126. Cette languette qui est représentée comprimée entre le corps de l'organe d'obturation et les parois intérieures du col de l'ampoule, est conçue de telle manière qu'elle se redresse, lorsque l'organe d'obturation pénètre dans la partie élargie de l'ampoule, et assure l'étanchéité entre le corps de l'organe d'obturation et les parois intérieures de l'ampoule, afin d'éviter que le liquide à injecter puisse s'échapper entre cet organe d'obturation et les parois de cette ampoule. Grâce à cette languette qui peut être remplacée par un bourrelet élastique compressible, tout le liquide médicamenteux s'écoule obligatoirement à travers le canal radial de l'organe d'obturation.

Comme le montre la fig. 13, au moment où l'opérateur dégage le bord supérieur 132 du col 121 de l'ampoule 120 hors des encoches 131, l'organe d'obturation 122 est aspiré vers le haut par l'effet du vide ménagé à l'intérieur de l'ampoule au moment du remplissage. Ce phénomène a pour effet de dégager les orifices latéraux de la branche transversale 126 et de permettre l'écoulement du médicament à travers la branche axiale 127 en direction du conduit traversant l'embout porte-aiguille 134. Pour vider l'ampoule, il suffit à l'opérateur de pousser le fond 135 de cette dernière en direction des ailettes latérales 130 ou inversement de tirer les ailettes 130 en direction du fond de l'ampoule 135.

L'organe d'obturation 122 peut être couplé à la tige de piston 125 par un vissage comme le montrent les figures. Toutefois, tout autre mode d'assemblage tel que par exemple un accrochage au moyen de becs ou d'un bourrelet s'engageant dans des encoches ou une rainure, pourrait également être utilisé à cet effet.

La fig. 14 représente une ampoule-seringue préremplie dont l'ampoule ou carpule 140 contient un liquide médicamenteux 141 et un gaz 142. Ce gaz se trouve à la pression atmosphérique ou à une pression légèrement supérieure obtenue au moment de la mise en place de l'organe d'obturation 143 qui comporte une partie supérieure cylindrique 144 munie d'un bourrelet périphérique 145 pourvu d'une entaille 146 permettant l'écoulement du liquide 141 lorsque l'organe d'obturation se trouve dans sa position d'utilisation et une partie inférieure 147. Cette partie inférieure est destinée d'une part à assurer le couplage de l'organe d'obturation avec la tige de piston 148 et d'autre part à assurer l'étanchéité entre cet organe d'obturation et les parois de l'ampoule lorsqu'il est repoussé à l'intérieur de cette ampoule. Pour assurer ce couplage de l'organe d'obturation 143 et de la tige de piston 148, la partie inférieure 147 de l'organe d'obturation comporte une zone rétrécie 154 pourvue d'une gorge 155 dans laquelle s'engage une protubérance 156 ménagée sur la tige de piston. Pour assurer l'étanchéité entre les parois de l'ampoule, dans sa zone large et l'organe d'obturation, ce dernier comporte un bourrelet 157, représenté écrasé du fait qu'il est localisé dans le col de diamètre réduit, et dont la fonction est identique à celle de la languette 133 du système des figures 12 et 13. Dans ce cas, comme dans l'exemple illustré par les figures 12 et 13, l'organe d'obturation joue le rôle d'un piston destiné à refouler le liquide hors de la carpule. Comme précédemment, l'organe d'obturation 143 comporte un conduit central 149 dont la branche axiale est raccordée au conduit 150 traversant axialement la tige du piston 148. La capsule 151 est dans ce cas composée de la tige de piston 148 et d'une douille 152 solidaire de cette tige de piston. Le rebord supérieur de cette douille 152 comporte une couronne 153 remplaçant les ailettes de préhension de la réalisation selon les figures 12 et 13.

L'exemple représenté par la fig. 15 constitue une variante de celui de la figure précédente. L'organe d'obturation 160 est réalisé d'une pièce avec la tige de piston. En vue du couplage de l'organe d'obturation 160 et de la capsule 161, cette dernière comporte à sa base, une gorge annulaire 162 dans laquelle est engagé un bourrelet ou rebord 163 ménagé au voisinage de la base de l'organe d'obturation 160 en dessous d'un bourrelet de retenue 164. Les autres éléments sont sensiblement similaires à ceux qui composent le dispositif représenté par la fig. 14. Toutefois, le bourrelet d'étanchéité 157 de l'organe d'obturation 143 est remplacé par une languette d'étanchéité 165 similaire à celle du dispositif des figures 12 et 13.

La fig. 16 illustre une autre variante selon laquelle l'organe d'obturation 170 est indépendant de la tige de piston 171 solidaire de la capsule 172. Entre l'extrémité supérieure de la tige de piston 171 et l'organe d'obturation se trouve une bague d'étanchéité 173. Dans cet exemple, la branche transversale 174 du conduit intérieur 175 ne comporte qu'une branche et est ménagée entre la bague 173 et l'organe d'obturation 170. L'avantage de ce dispositif réside dans le fait que la carpule préremplie peut être stockée indépendamment de la tige de piston solidaire de la capsule. Pour amener l'ampoule-seringue en position d'utilisation, il suffit de repousser l'organe d'obturation a l'intérieur de l'ampoule ou carpule 176 à l'aide de la tige de piston 171 en agissant sur les ailettes 177 et sur le fond 178 de l'ampoule.

Les figures 17 et 18 illustrent une variante particulièrement avantageuse dans certains cas, et spécifiquement réservée à des injections sous-cutanées à faibles volumes tels que l'insuline, la morphine, l'héparine, etc. L'ampoule 180 présente un col 181 de longueur relativement importante dont le volume intérieur correspond au volume de médicament 182 à injecter. L'organe d'obturation 183 obture l'extrémité ouverte du col 181 et le médicament est surmonté d'un gaz 184 détendu ou en très légère surpression. La fig. 17 illustre la position relative des éléments pendant le stockage, la tige de piston 185 solidaire ou non de la capsule 186 qui peut se présenter sous la forme d'une douille cylindrique ou comporter deux bras latéraux munis d'ailettes 187, pouvant être prémontée sur l'organe d'obturation 183 ou stockée séparément. La fig. 18 illustre la position d'utilisation. Au cours de cette phase, l'organe d'obturation 183 a été enfoncé dans le col 181 de l'ampoule, de sorte que le médicament soit remonté dans le corps de cette ampoule de manière à comprimer fortement le gaz 184. Après cette opération préliminaire, l'ampoule-seringue décrite est utilisable d'une manière sensiblement équivalente à celles des ampoules à gaz sous pression décrites précédemment. Le volume de l'ampoule doit être suffisant pour contenir le volume de liquide à injecter plus le gaz comprimé par la réduction de volume induite par l'avance du piston entre ses positions d'obturation et de débit. Il est possible de varier le volume de l'ampoule en fonction de la force maximale que l'on désire appliquer sur le piston. Cette force dépendra de la réduction de volume que doit subir le gaz. Une réduction de 50 % impose une pression de 1 bar. Une réduction de 50 % permet de chasser la même quantité de liquide que la réduction de volume subie par le gaz. A titre d'exemple, si le liquide à injecter est de 0,5 ml., le volume gagné par la compression du gaz doit être d'au moins 0,5 ml., ce qui détermine la longueur du col de l'ampoule. La longueur du col est calculée précisément pour que la réduction de volume occupée par le gaz entre la position d'obturation et la position de débit, de l'organe d'obturation induise une pression suffisante pour que le gaz comprimé par la réduction de volume, puisse en se dilatant chasser le liquide. Une légère surpression induite par la mise en place de l'organe d'obturation devrait être suffisante pour chasser le liquide hors du conduit. L'espace réservé pour le gaz comprimé pourrait être réduit en théorie à l'espace que ce gaz pourrait occuper à son stade d'incompressibilité. Il faudrait dans ce cas appliquer une force importante. Il est plus intéressant de réserver assez de place au gaz après réduction de volume pour que la force à appliquer soit relativement faible. Si l'on désire obtenir une réduction de volume du gaz plus importante que le volume du liquide à injecter, il suffit de rendre le volume du col supérieur au volume de liquide à injecter. On peut ainsi augmenter la rapidité de débit et vaincre plus facilement la résistance de la pénétration de liquide dans la peau du patient. Mais alors on court le risque d'injecter un peu de gaz en fin d'injection, ce qui n'est en fait pas dangereux en soi.

Pour remédier à cela, on a équipé le fond de la tige de piston d'un joint compressible 188 en fin de course de l'ampoule en position de débit. Il suffit à l'infirmière d'observer la fin d'écoulement du liquide et de relâcher sa pression sur l'ampoule. Ce mouvement permettra au joint de se détendre et de faire reculer d'un mm environ l'ampoule pour l'amener en position d'obturation et interrompre le débit de gaz.

Les figures 19 et 20 illustrent une autre forme de réalisation dans laquelle l'organe d'obturation 200 comporte à sa base un prolongement cylindrique 201, fixé, par exemple par vissage dans une embase 202 solidaire de la capsule 203 comportant une douille extérieure 204 dont le bord supérieur porte les ailettes 205. Le conduit central 206 de l'organe d'obturation se compose comme précédemment d'un canal axial et d'un canal radial disposé à l'extrémité supérieure du canal axial. A l'intérieur de ce dernier, est logé un conduit 207 dont l'extrémité supérieure est libre et dont l'extrémité inférieure est raccordée au fond de la cavité intérieure de la capsule. Dans la position de repos illustrée par la fig. 19, l'extrémité libre du conduit 207 neutralise la communication entre le canal radial et le canal axial du conduit 207. Pour amener l'ampoule-seringue dans sa position d'utilisation, l'opérateur dévisse l'embase 202 du prolongement cylindrique 201 en tournant la capsule 203 par rapport à l'ampoule préremplie 208. Ceci a pour effet de déplacer axialement vers le bas le conduit 207, de telle manière que la communication entre le canal axial et le canal radial du conduit central 206 soit rétablie. Toutefois, dans cette position intermédiaire les orifices du conduit radial restent obturés parce qu'ils sont directement en contact avec le col de l'ampoule, et en raison de l'existence du bourrelet qui coiffe l'extrémité supérieure de l'organe d'obturation 200. Pour amener le dispositif dans sa position d'utilisation, l'opérateur pousse sur le fond de l'ampoule en tirant sur les ailettes 205 et amène ainsi l'organe d'obturation 200, faisant office de piston dans le corps de l'ampoule dont le diamètre est supérieur à celui du col. L'étanchéité entre le corps de l'ampoule et l'organe d'obturation reste, dans cette position, assurée par le bourrelet 209 représenté comprimé sur la fig. 19 et détendu sur la fig. 20.

Ce dispositif télescopique est avantageux du fait qu'il autorise un stockage sous un encombrement relativement réduit.

Les figures 21, 22 et 23 illustrent une ampoule-seringue à conduit intérieur télescopique basé sur les mêmes principes que ceux de la réalisation précédente. Toutefois, dans ce cas le canal 210 logé à l'intérieur de la branche axiale du conduit intérieur 211 en forme de T de l'organe d'obturation 212 est solidaire d'une pièce cylindrique 213 pourvue de deux protubérances 214 en forme de secteurs angulaires diamétralement opposés (voir en particulier la fig. 23) et qui est logée à l'intérieur d'une cavité cylindrique 215 ménagée à la base de l'organe d'obturation 212. Ce système à baïonnette remplit les mêmes fonctions que le système d'accouplement vissé de la réalisation précédente. Une rotation d'un quart de tour suivie d'un déplacement axial vers le bas de la capsule 216 par rapport à l'ampoule 217 permet de désobturer le conduit intérieur 211. Par la suite, l'ampoule-seringue est prête à l'emploi comme décrit en référence aux figures 19 et 20.

La fig. 24 illustre un mode de montage de la tige de piston 220, solidaire de la capsule 221 munie d'ailettes 222, sur l'organe d'obturation 223. A cet effet, l'organe d'obturation 223 comporte un évidement 224 dans lequel s'engage un embout 225 qui sert à coupler ledit organe d'obturation avec ladite tige de piston. Le conduit intérieur 226 est décentré, de sorte que, contrairement aux autres réalisations, la capsule ne présente pas de symétrie axiale et l'embout porte-aiguille 227 est excentré. La branche transversale 228 communique avec la branche axiale du conduit intérieur 226. Une telle réalisation asymétrique est particulièrement utilisée lorsque les volumes de médicaments à injecter sont importants. Comme mentionné précédemment, la languette 229 qui est représentée sur cette réalisation, pourrait être remplacée par un bourrelet compressible.

La fig. 25 illustre une forme de réalisation particulièrement avantageuse d'une ampoule 230 comportant un étranglement annulaire 231 qui divise l'intérieur de cette ampoule en deux chambres 232 et 233 contenant respectivement le liquide médicamenteux et le gaz sous pression. En raison de phénomènes de tension superficielle du liquide médicamenteux particulièrement visqueux, le liquide reste constamment dans la chambre inférieure 232 et ne peut pas pénétrer dans la chambre supérieure 233, quelle que soit la position de cette ampoule pendant son stockage et/ou son transport. Il en résulte que le liquide est toujours en contact avec l'organe d'obturation, ce qui empêche un contact direct du gaz sous pression avec cet organe et garantit le maintien sous pression du liquide, en excluant toute possibilité de fuite de ce gaz. Bien que l'étranglement 231 soit localisé approximativement aux 2/3 supérieurs de l'ampoule, il peut également se trouver en un endroit quelconque, par exemple au 1/3 inférieur, comme représenté par des traits interrompus en 231'. Dans ce cas, la chambre inférieure localisée entre l'étranglement 231' et l'organe d'obturation (non représenté) contient du liquide médicamenteux qui ne peut s'échapper vers la chambre supérieure. De ce fait, l'extrémité de l'organe d'obturation intérieure a l'ampoule est constamment baignée par le liquide et n'entre jamais en contact avec le gaz sous pression, quelle que soit la position de l'ampoule. La chambre supérieure, par contre, contient du liquide et de l'air dont la position relative dépend de la position de l'ampoule. Lors de l'injection, l'ampoule est tenue verticalement de sorte que l'air comprimé chasse le liquide de la chambre supérieure dans la chambre inférieure puis à travers l'aiguille dans le corps du patient. L'étranglement peut être réalisé directement par déformation des parois de l'ampoule ou être obtenu par une bague rapportée à l'intérieur de cette ampoule.

Les nombreuses formes de réalisation décrites ci-dessus présentent toutes l'avantage d'une grande simplicité de fabrication. Les pièces se prêtent particulièrement bien à une réalisation en matière synthétique injectée et peuvent ensuite être assemblées de manière automatique. Le remplissage peut être réalisé économiquement par des appareils usuels et dans des conditions d'hygiène exceptionnelles. L'utilisation n'offre aucune possibilité aux erreurs de manipulation et les opérations préparatoires en vue de l'utilisation n'engendrent aucun déchet solide dangereux pour le patient, tel que débris de verre dus à la cassure d'une ampoule ou particules de matière synthétique provenant du perçage d'un obturateur au moyen d'une aiguille ou d'un autre dispositif similaire. L'ampoule-seringue selon l'invention répond à la fois aux exigences économiques et aux critères de sécurité recherchés à la fois par les fabricants et les utilisateurs.

**Revendications**

1. Ampoule-seringue comprenant une ampoule (1, 100, 120, 140, 176, 180, 208, 217, 230) préremplie d'un liquide, notamment d'un médicament à injecter, ladite ampoule étant pourvue à son extrémité ouverte d'un organe d'obturation (5, 27, 40, 52, 60, 73, 91, 115, 122, 143, 160, 170, 183, 200, 212, 223) et de distribution mobile axialement par rapport à l'ampoule entre une première position dite d'obturation et une seconde position dite d'injection, cet organe comportant à son extrémité externe un embout porte-aiguille (9, 37, 75, 98, 134, 227) et étant traversé par un conduit (7, 28, 44, 54, 90, 149, 175, 206, 211, 226) reliant ledit embout à l'intérieur de l'ampoule lorsque ledit organe se trouve dans sa position d'injection, ledit conduit comprenant une branche axiale (10, 43, 34, 126, 174, 211) débouchant à l'extérieur du côté de l'embout porte-aiguille et une branche transversale (10, 34, 43, 126, 174, 228) communiquant avec ladite branche axiale, la ou les extrémités ouvertes de ladite branche transversale débouchant à l'intérieur de l'ampoule, caractérisée en ce que ladite ampoule présente à son extrémité ouverte un col (2, 41, 50, 64, 71, 93, 121, 181) de section rétrécie par rapport à son corps, en ce que ledit organe d'obturation est disposé à l'intérieur dudit col en contact direct avec sa paroi interne, cet organe ayant une forme sensiblement complémentaire et des dimensions sensiblement égales à celles dudit col, et en ce que la branche transversale dudit conduit est ménagée dans l'organe d'obturation en position telle que son ou ses extrémités débouchant à l'intérieur de l'ampoule soient obturées par la paroi interne du col de l'ampoule lorsque l'organe d'obturation se trouve dans ladite position d'obturation.

2. Ampoule-seringue selon la revendication 1, caractérisée en ce que l'organe d'obturation comporte à son extrémité en contact avec l'intérieur de l'ampoule, un bourrelet périphérique élastique (42, 84, 115) dont le diamètre externe est supérieur au plus petit diamètre interne du col de cette ampoule, et en ce que ce bourrelet est muni d'au moins une entaille périphérique (45, 85, 146) dont la profondeur est sensiblement égale à la largeur dudit bourrelet.

3. Ampoule-seringue selon la revendication 1, caractérisée en ce que l'organe d'obturation (40) présente une forme tronconique sensiblement complémentaire à la forme tronconique du col (41) de l'ampoule.

4. Ampoule-seringue selon la revendication 1, caractérisée en ce que l'organe d'obturation comporte au moins une rainure et au moins un bourrelet contigu (19), ménagés à la périphérie de cet organe en dessous de la branche transversale du conduit central (Fig. 2).

5. Ampoule-seringue selon la revendication 1, caractérisée en ce que l'organe d'obturation (52) comporte sur sa paroi externe au moins une portion périphérique agencée pour constituer une butée élastique pour une protubérance interne (51) solidaire du col de l'ampoule (1) (Fig. 5).

6. Ampoule-seringue selon la revendication 5, caractérisée en ce que la portion périphérique est constituée par un bourrelet ménagé à la périphérie de l'organe d'obturation (Fig. 5).

7. Ampoule-seringue selon la revendication 1, caractérisée en ce que l'organe d'obturation comporte une protubérance annulaire (133, 157, 229) disposée en dessous de l'orifice ou des orifices de ladite branche transversale (126, 228) du conduit, cette protubérance étant compressible de telle manière qu'elle soit en appui contre les parois du col de l'ampoule lorsqu'elle se trouve localisée, à l'état comprimé dans ladite position d'obturation, à l'intérieur du col et qu'elle soit en appui contre les parois du corps de l'ampoule lorsqu'elle se trouve localisée, à l'état détendu dans ladite position d'injection, à l'intérieur du corps de cette ampoule (Fig. 12, 13, 14, 24).

8. Ampoule-seringue selon la revendication 7, caractérisée en ce que la protubérance est constituée par une languette souple.

9. Ampoule-seringue selon la revendication 1, caractérisée en ce que le col de l'ampoule comporte un bourrelet continu ou discontinu (3, 23, 63, 72, 96) ménagé à l'intérieur ou à l'extérieur dudit col de l'ampoule.

10. Ampoule-seringue selon la revendication 1, caractérisée en ce que l'organe d'obturation est solidaire d'une capsule (4, 25, 74, 92, 112, 151, 161, 172, 186, 209, 221) pourvue d'un évidement central, conçue pour s'adapter au moins en partie autour du col de l'ampoule, et en ce que l'organe d'obturation est fixé à l'intérieur de cette capsule coaxialement avec son évidement central.

11. Ampoule-seringue selon la revendication 10, caractérisée en ce que la capsule présente un rebord évasé (13, 66, 76, 110, 130, 153, 177, 205, 222) au voisinage de son extrémité comportant l'embout porte-aiguille.

12. Ampoule-seringue selon les revendications 9 et 10, caractérisée en ce que la capsule comporte au moins un bourrelet de retenue (14, 24, 65, 82) agencé pour coopérer avec ledit bourrelet continu ou discontinu de l'ampoule pour retenir la capsule en position sur l'ampoule.

13. Ampoule-seringue selon les revendications 9 et 10, caractérisée en ce que la capsule comporte au moins deux bras latéraux munis d'ailettes (13, 66, 76, 110, 130, 153, 177, 205, 222) et pourvus d'organes de retenue qui coopèrent avec ledit bourrelet continu ou discontinu de l'ampoule pour constituer des butées définissant la position d'obturation de l'organe d'obturation solidaire de cette capsule.

14. Ampoule-seringue selon la revendication 10, caractérisée en ce que l'organe d'obturation et la capsule comportent des moyens d'accouplement (29, 30 ; 79, 80 ; 91, 94 ; 155, 156 ; 163, 164 ; 201, 202 ; 213, 214) conçus pour fixer ledit organe d'obturation à l'intérieur de ladite capsule.

15. Ampoule-seringue selon la revendication 10, caractérisée en ce que l'organe d'obturation est solidaire d'une tige de piston (125, 148, 171, 185) solidaire de la capsule (Fig. 12, 13, 14, 15, 16, 17, 18).

16. Ampoule-seringue selon la revendication 15, caractérisée en ce que la capsule comporte deux bras allongés (129, 152), pourvus d'ailettes latérales (130, 153) et d'organes de verrouillage (131, 132) coopérant avec le bord de l'ampoule pour bloquer l'organe d'obturation en position d'obturation pendant le stockage (Fig. 12, 13, 14, 15).

17. Ampoule-seringue selon la revendication 15, caractérisée en ce que la tige de piston est réalisée d'une pièce avec l'organe d'obturation.

18. Ampoule-seringue selon la revendication 15, caractérisée en ce que la tige de piston accouplée à l'organe d'obturation a une longueur utile au moins égale à la longueur intérieure de l'ampoule.

19. Ampoule-seringue selon la revendication 10, caractérisée en ce que l'ampoule (180) a un col (181) dont la longueur est telle que le déplacement de l'organe d'obturation à l'intérieur de ce col pour atteindre ladite position d'injection, induit dans le corps de l'ampoule une surpression suffisante pour assurer l'évacuation complète du liquide (Fig. 17, 18).

20. Ampoule-seringue selon la revendication 10, caractérisée en ce que la capsule comporte une partie tubulaire destinée à déplacer à l'intérieur de la branche axiale du conduit de l'organe d'obturation, cette partie tubulaire étant agencée pour obstruer la branche transversale dudit conduit lorsque l'organe d'obturation se trouve dans ladite position d'obturation et pour libérer le passage entre cette branche transversale et cette branche axiale lorsque l'organe d'obturation se trouve dans ladite position d'injection (Fig. 19, 20, 21, 22).

21. Ampoule-seringue selon la revendication 20, caractérisée en ce que la capsule et l'organe d'obturation comportent des moyens (201, 202, 213, 215) qui coopèrent pour assurer un déplacement relatif d'une part de l'organe d'obturation et de l'ampoule dans leurs positions relatives d'obturation, et d'autre part de la capsule pour amener cette dernière dans ladite position d'injection (Fig. 19, 20, 21, 22).

22. Ampoule-seringue selon la revendication 21, caractérisée en ce que lesdits moyens (201, 202) comportent une embase filetée (202) solidaire de la capsule et un prolongement cylindrique fileté (201) solidaire de l'organe d'obturation.

23. Ampoule-seringue selon la revendication 21, caractérisée en ce que lesdits moyens (213, 215) comportent une pièce cylindrique (213) munie de deux protubérances (214) en forme de secteurs angulaires, solidaires de la capsule et un évidement sensiblement cylindrique (215) ménagé dans l'organe d'obturation, les deux éléments réalisant un couplage du type à baïonnette.

24. Ampoule-seringue selon la revendication 1, caractérisée en ce que l'ampoule (230) comporte un étranglement annulaire (231) conçu pour diviser l'espace intérieur de cette ampoule en deux chambres (232, 233) dont celle, délimitée à une extrémité par l'organe d'obturation ne peut contenir que du liquide, quelle que soit la position de l'ampoule.

**Claims**

1. Ampoule-syringe comprising an ampoule (1, 100, 120, 140, 176, 180, 208, 217, 230) prefilled with a liquid, especially a liquid medication to be injected, said ampoule being provided, at its open end, with stopper and distribution means (5, 27, 40, 52, 60, 73, 91, 115, 122, 143, 160, 170, 183, 200, 212, 223) which is axially movable with respect to the ampoule, between a first position called stopping position and a second position called injection position said stopper and distribution means comprising, at its outside end, a needleholding tip (9, 37, 75, 98, 134, 227) and being provided with a conduit (7, 28, 44, 54, 90, 149, 175, 206, 211, 226) connecting said needle-holding tip to the interior of the ampoule, when said stopper means is in its injection position, said conduit comprising an axial branch (10, 43, 34, 126, 174, 211) opening directly outside of the needle-holding tip and a radial branch (10, 34, 43, 126, 174, 228) connected to said axial branch, one or the two open ends of said radial branch opening directly inside the ampoule, characterized in that said ampoule is provided, at its open end, with a neck (2, 41, 50, 64, 71, 93, 121, 181) having a reduced section with respect to the body, in that said stopper means is located inside said neck, in contact with its internal walls, said stopper means having a substantially complementary shape and equal dimensions as said neck and in that the radial branch of said conduit is disposed in the stopper means in such a manner that one or both of its ends opening directly inside the ampoule are stoppered by the internal walls of the ampoule neck when the stopper means is in its first position.

2. Ampoule-syringe according to claim 1, characterized in that the stopper means comprises at the end in contact with the interior of the ampoule, a peripheral elastic rim (42, 84, 115) having an external diameter greater than the smallest internal diameter of the ampoule neck, and in that said rim is provided with at least one peripheral groove (45, 85, 146) of a depth essentially equal to the breadth of the said rim.

3. Ampoule-syringe according to claim 1, characterized in that the stopper means (40) is of truncated shape essentially complementary to the truncated shape of the ampoule neck (41).

4. Ampoule-syringe according to claim 1, characterized in that the stopper means comprises at least one groove and at least one rim (19) disposed on the periphery of said element beneath the radial branch of the central conduit (Fig. 2).

5. Ampoule-syringe according to claim 1, characterized in that the stopper means (52) comprises on its external walls, at least one peripheral part disposed to constitute an elastic stop for an internal protuberance (51) integral with the ampoule neck (Fig. 5).

6. Ampoule-syringe according to claim 5, characterized in that the peripheral part is comprised of a rim, disposed on the periphery of the stopper means.

7. Ampoule-syringe according to claim 1, characterized in that the stopper means comprises an annular protuberance (133, 157, 299) disposed beneath one or the orifices of the radial branch (126, 228) of the conduit, said protuberance being compressible so that it is in contact with the walls of the ampoule neck when the protuberance is compressed and positioned inside the neck, and so that it is in contact with the walls of the ampoule body when positioned, in its expanded state, inside said ampoule body (Fig. 12, 13, 14, 24).

8. Ampoule-syringe according to claim 7, characterized in that the protuberance has the form of a flexible lip.

9. Ampoule-syringe according to claim 1, characterized in that the ampoule neck comprises a continuous or discontinuous rim (3, 23, 63, 72, 96) disposed inside or outside the said ampoule neck.

10. Ampoule-syringe according to claim 1, characterized in that the stopper means is integral with a capsule (4, 25, 74, 92, 112, 151, 161, 172, 186, 209, 221) provided with a central opening, designed to at least partially adapt with the ampoule neck, and in that the stopper means is affixed inside said capsule in a coaxial manner with its central opening.

11. Ampoule-syringe according to claim 10, characterized in that the capsule has a raised edge (13, 66, 76, 110, 130, 153, 177, 205, 222) with the end comprising the needle-holding tip.

12. Ampoule-syringe according to claims 9 and 10, characterized in that the capsule comprises at least one retaining rim (14, 24, 65, 82) disposed to cooperate with said continuous or discontinuous rim of the ampoule to maintain the capsule in position on the ampoule.

13. Ampoule-syringe according to claim 9 and 10, characterized in that the capsule comprises at least two lateral arms provided with projections (13, 66, 76, 110, 130, 153, 177, 205, 222) having retaining means which cooperate with said continuous or discontinuous rim of the ampoule neck to comprise stops defining the stopping position of the stopper means integral with said capsule.

14. Ampoule-syringe according to claim 10, characterized in that the stopper means and the capsule comprise coupling means (29, 30 ; 79, 80 ; 91, 94 ; 155, 156 ; 163, 164 ; 201, 202 ; 213, 214) designed to affix the said stopper means inside of the said capsule.

15. Ampoule-syringe according to claim 10, characterized in that the stopper means is integral with a piston stem (125, 148, 171, 185) integral with the capsule (Fig. 12, 13, 14, 15, 16, 17, 18).

16. Ampoule-syringe according to claim 15, characterized in that the capsule comprises two elongated arms (129, 152) provided with lateral projections (130, 153) and locking means (131, 132) which cooperate with the rim of the ampoule to block the stopper means in stopped position during storage (Fig. 12, 13, 14, 15).

17. Ampoule-syringe according to claim 15, characterized in that the piston stem is of one piece with the stopper means.

18. Ampoule-syringe according to claim 15, characterized in that the piston stem connected to the stopper means is at least equal in length to the internal length of the ampoule.

19. Ampoule-syringe according to claim 10, characterized in that the ampoule (180) has a neck (181) which length is such that the displacement of the stopper means inside said neck to reach said injection position, induces in the body of the ampoule, an overpressure sufficient to insure the complete evacuation of the liquid (Fig. 17, 18).

20. Ampoule-syringe according to claim 10, characterized in that the capsule comprises a cylindrical portion to be moved inside of the axial branch of the conduit of the stopper means, this cylindrical portion being disposed to obstruct the radial branch of said conduit when the stopper means is in said stopping position and to provide free passage between this radial branch and this axial branch when the stopper means is in said injection position (Fig. 19, 20, 21, 22).

21. Ampoule-syringe according to claim 20, characterized in that the capsule and the stopper means comprise means (201, 202, 213, 215) for cooperation ensuring the relative displacement on the one hand of the stopper means and the ampoule when in their respective stopping positions, and on the other hand of the capsule to place it in its injection position (Fig. 19, 20, 21, 22).

22. Ampoule-syringe according to claim 21, characterized in that the said means (201, 202) comprise a threaded base (202) integral with the capsule and a threaded cylindrical extension (201) integral with the stopper means.

23. Ampoule-syringe according to claim 21, characterized in that the said means (213, 215) comprise a cylindrical portion (213) provided with two protuberances (214) in the form of angular segments, integral with the capsule and an essentially cylindrical opening (215) disposed in the stopper means, said two elements effecting a bayonet-like coupling action.

24. Ampoule-syringe according to claim 1, characterized in that the ampoule (230) has an annular constriction (231) dividing its interior into two chambers, (232, 233) one of which, the chamber adjacent to the stopper means, can contain only a liquid, regardless of the position of the ampoule.

**Patentansprüche**

1. Ampullenspritze bestehend aus einer mit Flüssigkeit, insbesondere mit einem zu injizierenden Medikament, vorgefüllten Ampulle (1, 100, 120, 140, 176, 180, 208, 217, 230), wobei besagte Ampulle an ihrem offenen Ende mit einem Organ (5, 27, 40, 52, 60, 73, 91, 115, 122, 143, 160, 170, 183, 200, 212, 223) zum Verschließen und zum Ausspritzen, welches bezüglich der Ampulle zwischen einer ersten Position, mit Verschlußposition bezeichnet, und einer zweiten Position, mit Injektionsposition bezeichnet, axial beweglich ist, wobei dieses Organ an seinem äußeren Ende einen Ansatz (9, 37, 75, 98, 134, 227) zum Aufnehmen einer Nadel aufweist und welches von einem Kanal (7, 28, 44, 54, 90, 149, 175, 206, 211, 226) durchbrochen ist, der besagten Ansatz mit dem Inneren der Ampulle verbindet, wenn sich besagtes Organ in der Injektionsposition befindet, wobei besagter Kanal ein Längsstück (10, 43, 34, 126, 174, 211) aufweist, welches außen am Nadelträgeransatz mündet, und ein Querstück (10, 34, 43, 126, 174, 228), welches mit besagtem Längsstück in Verbindung steht, wobei das oder die offenen Enden des besagten Querstückes im Inneren der Ampulle münden, dadurch gekennzeichnet, daß besagte Ampulle an ihrem offenen Ende einen Hals (2, 41, 50, 64, 71, 93, 121, 181) mit einem Querschnitt aufweist, der bezüglich ihres Schaftes eingeschnürt ist, daß besagtes Verschlußorgan im Inneren des besagten Halses in direktem Kontakt mit seiner Innenwand angeordnet ist, wobei dieses Organ im wesentlichen gleichförmig und in den Dimensionen im wesentlichen gleich denen des besagten Halses ist, und daß das Querstück des besagten Kanales in dem Verschlußorgan derart angebracht ist, daß sein oder seine Enden, die im Inneren der Ampulle münden, durch die innere Wand des Ampullenhalses verschlossen sind, wenn sich das Verschlußorgan in besagter Verschlußposition befindet.

2. Ampullenspritze nach Anspruch 1, dadurch gekennzeichnet, daß das Verschlußorgan an seinem Ende, in Kontakt mit dem Inneren der Ampulle, einen elastischen Umfangswulst (42, 84, 115) aufweist, dessen äußerer Durchmesser größer als der kleinste innere Durchmesser des Halses dieser Ampulle ist, und daß dieser Wulst mit mindestens einer Nut (45, 85, 146) am Umfang versehen ist, deren Tiefe etwa gleich der Dicke des besagten Wulstes ist.

3. Ampullenspritze nach Anspruch 1, dadurch gekennzeichnet, daß das Verschlußorgan (40) eine kegelstumpfähnliche Form darstellt, die im wesentlichen die gleiche Form wie der kegelstumpfähnliche Hals (41) der Ampulle hat.

4. Ampullenspritze nach Anspruch 1, dadurch gekennzeichnet, daß das Verschlußorgan mindestens eine Rille und mindestens einen Nebenwulst (19) aufweist, die auf der Außenseite dieses Organes unterhalb des Querstückes des besagten zentralen Kanales angebracht sind.

5. Ampullenspritze nach Anspruch 1, dadurch gekennzeichnet, daß das Verschlußorgan (52) auf seiner Außenwand mindestens einen Umfangsbereich aufweist, um einen elastischen Anschlag für einen mit dem Hals der Ampulle (1) (Figur 5) fest verbundenen inneren Vorsprung (51) zu bilden.

6. Ampullenspritze nach Anspruch 5, dadurch gekennzeichnet, daß der Umfangsbereich durch einen Wulst gebildet ist, der am Umfang des Verschlußorganes (Figur 5) angebracht ist.

7. Ampullenspritze nach Anspruch 1, dadurch gekennzeichnet, daß das Verschlußorgan einen ringförmigen Vorsprung (133, 157, 229) aufweist, der unterhalb der Mündung oder der Mündungen des besagten Kanalquerstückes (126, 228) liegt, wobei dieser Vorsprung so zusammendrückbar ist, daß er sich gegen die Wände des Ampullenhalses legt, wenn er sich im zusammengedrückten Zustand in der besagten Verschlußposition im Inneren des Halses befindet und daß er sich gegen die Wände des Ampullenschaftes legt, wenn er sich im entspannten Zustand in der besagten Injektionsposition im Inneren des Schaftes dieser Ampulle (Figur 12, 13, 14, 24) befindet.

8. Ampullenspritze nach Anspruch 7, dadurch gekennzeichnet, daß der Vorsprung als weiche Zunge ausgebildet ist.

9. Ampullenspritze nach Anspruch 1, dadurch gekennzeichnet, daß der Ampullenhals einen durchlaufenden oder unterbrochenen Wulst (3, 23, 63, 72, 96) aufweist, der innen oder außen an dem besagten Ampullenhals angeordnet ist.

10. Ampullenspritze nach Anspruch 1, dadurch gekennzeichnet, daß das Verschlußorgan mit einer Kapsel (4, 25, 74, 92, 112, 151, 161, 172, 186, 209, 221) fest verbunden ist, die eine zentrale Aussparung aufweist, damit sie sich wenigstens teilweise um den Ampullenhals anpaßt, und daß das Verschlußorgan im Inneren dieser Kapsel koaxial zu dessen zentraler Aussparung befestigt ist.

11. Ampullenspritze nach Anspruch 10, dadurch gekennzeichnet, daß die Kapsel in der Nähe ihres Endes wo sich der Nadelträgeransatz befindet einen ausladenden Rand (13, 66, 76, 110, 130, 153, 177, 205, 222) aufweist.

12. Ampullenspritze nach den Ansprüchen 9 und 10, dadurch gekennzeichnet, daß die Kapsel mindestens einen Rückhaltewulst (14, 24, 65, 82) aufweist, der vorgesehen ist, um mit dem besagten durchlaufenden oder unterbrochenen Wulst der Ampulle zusammenzuwirken, um die Kapsel auf der Ampulle festzuhalten.

13. Ampullenspritze nach den Ansprüchen 9 und 10, dadurch gekennzeichnet, daß die Kapsel mindestens zwei seitliche mit Flügeln versehene Ansätze (13, 66, 76, 110, 130, 153, 177, 205, 222) aufweist und mit Rückhalteorganen versehen ist, welche mit dem besagten durchlaufenden oder unterbrochenen Wulst der Ampulle zusammenwirken, um Anschläge zu bilden, welche die Verschlußposition des mit dieser Kapsel fest verbundenen Verschlußorganes definieren.

14. Ampullenspritze nach Anspruch 10, dadurch gekennzeichnet, daß das Verschlußorgan und die Kapsel Kupplungsmittel (29, 30 ; 79, 80 ; 91, 94 ; 155, 156 ; 163, 164 ; 201, 202 ; 213, 214) aufweist, die zum Fixieren des besagten Verschlußorganes im Inneren der besagten Kapsel dienen.

15. Ampullenspritze nach Anspruch 10, dadurch gekennzeichnet, daß das Verschlußorgan fest mit einer Kolbenstange (125, 148, 171, 185) verbunden ist, die ihrerseits mit der Kapsel fest verbunden ist (Fig. 12, 13, 14, 15, 16, 17, 18).

16. Ampullenspritze nach Anspruch 15, dadurch gekennzeichnet, daß die Kapsel zwei längliche Arme (129, 152) aufweist, die mit seitlichen Flügeln (130, 153) und mit Blockierungsorganen (131, 132) versehen sind, die mit dem Ampullenrand zusammenwirken, um das Verschlußorgan während der Lagerung in Verschlußstellung zu blockieren (Fig. 12, 13, 14, 15).

17. Ampullenspritze nach Anspruch 15, dadurch gekennzeichnet, daß die Kolbenstange und das Verschlußorgan aus einem Stück bestehen.

18. Ampullenspritze nach Anspruch 15, dadurch gekennzeichnet, daß die mit dem Verschlußorgan verbundene Kolbenstange eine nutzbare Länge aufweist, die mindestens gleich der inneren Länge der Ampulle ist.

19. Ampullenspritze nach Anspruch 10, dadurch gekennzeichnet, daß die Ampulle (180) einen Hals (181) hat, dessen Länge so gestaltet ist, daß die Verschiebung des Verschlußorganes im Inneren dieses Halses, um die besagte Injektionsposition zu erreichen, im Schaft der Ampulle einen Überdruck induziert, der zur vollständigen Entleerung der Flüssigkeit ausreicht (Figur 17, 18).

20. Ampullenspritze nach Anspruch 10, dadurch gekennzeichnet, daß die Kapsel ein rohrförmiges Teil aufweist, welches sich im Inneren des axialen Kanalstückes des Verschlußorganes verschieben kann, wobei dieses rohrförmige Teil dazu vorgesehen ist, das Querstück des besagten Kanales abzustellen, wenn sich das Verschlußorgan in der besagten Verschlußstellung befindet und um den Durchgang zwischen diesem Querstück und diesem axialen Stück freizugeben, wenn sich das Verschlußorgan in der besagten Injektionsposition befindet (Figur 19, 20, 21, 22).

21. Ampullenspritze nach Anspruch 20, dadurch gekennzeichnet, daß die kapsel und das Verschlußorgan Mittel (201, 202, 213, 215) aufweisen, die zusammenwirken, um eine relative Verschiebung zu gewährleisten, einerseits des Verschlußorganes und der Ampulle in ihre relativen Verschlußpositionen und andererseits der Kapsel, um letztere in die besagte Injektionsposition mitzunehmen (Figur 19, 20, 21, 22).

22. Ampullenspritze nach Anspruch 21, dadurch gekennzeichnet, daß die besagten Mittel (201, 202) einen mit Gewinde versehenen, mit der Kapsel fest verbundenen Sockel (202) und eine zulindrische, mit Gewinde versehene Verlängerung (201), die fest mit dem Verschlußorgan verbunden ist, aufweisen.

23. Ampullenspritze nach Anspruch 21, dadurch gekennzeichnet, daß die besagten Mittel (213, 215) ein zylindrisches Teil (213) aufweisen, das mit zwei fest mit der Kapsel verbundenen Vorsprüngen (214) in Form von Kreissegmenten versehen ist und eine im wesentlichen zylindrische Aussparung (215), die im Verschlußorgan angeordnet ist, wobei die zwei Elemente eine Bajonettkupplung darstellen.

24. Ampullenspritze nach Anspruch 1, dadurch gekennzeichnet, daß die Ampulle (230) eine ringförmige Einschnürung (231) aufweist, die vorgesehen ist, um den Innenraum dieser Ampulle in zwei Kammern (232, 233) aufzuteilen, wovon diejenige, die an einem Ende durch das Verschlußorgan begrenzt ist, nur Flüssigkeit enthalten kann, unabhängig von der Stellung der Ampulle.

FIG. 1

FIG. 2

FIG. 3

FIG. 4

FIG. 5

FIG. 6

**FIG. 7**

**FIG. 8**

**FIG. 9**

**FIG. 10**

**FIG. 11**

**FIG. 12**

FIG. 13     FIG. 14     FIG. 15

FIG. 16

FIG. 17

FIG. 18

FIG. 19

FIG. 20

FIG. 21

FIG. 25

FIG. 22

FIG. 23

FIG. 24